# EUROPÄISCHE PATENTSCHRIFT

(11) **EP 1 782 734 B1**
(45) Veröffentlichungstag und Bekanntmachung des Hinweises auf die Patenterteilung: **24.10.2018**
(21) Anmeldenummer: 06022848.3
(22) Anmeldetag: 02.11.2006
(51) Int. Cl.: A61B 6/12, A61B 6/00, A61B 90/00

(54) **Vorrichtung zur Verbesserung der Volumenrekonstruktion**
Device for improving volume reconstruction
Dispositif pour améliorer le reconstruction d'images tridimensionelles

(30) Priorität: 05.11.2005 DE 102005052787
(43) Veröffentlichungstag der Anmeldung: 09.05.2007
(73) Patentinhaber: Ziehm Imaging GmbH, 90451 Nürnberg (DE)
(72) Erfinder: Hörndler, Klaus, 90482 Nürnberg (DE); Fleischmann, Christof, 91096 Möhrendorf (DE); Kränzel, Wolfgang, 90766 Fürth (DE)

(56) Entgegenhaltungen:
- DE-A1- 10 202 091

## Beschreibung

Die Erfindung betrifft eine Vorrichtung zur Verfügungstellung einer verbesserten Rekonstruktion eines Volumens eines Untersuchungsobjektes unter Verwendung einer Röntgendiagnostikeinrichtung mit einem Bildverarbeitungsrechner, eines Lageerfassungssystems und eines Phantoms, das in einer festen räumlichen Beziehung röntgenpositive Marken und solche enthält, die von dem Lageerfassungssystem erfaßbar sind

Medizinische Eingriffe an lebenden Objekten werden zunehmend mittels Navigationsunterstützung durchgeführt. Darunter versteht man die mittels eines Lageerfassungssystems unterstützte Führung eines Instrumentes relativ zu einem in Behandlung stehenden Gewebebereichs des Objektes. Von besonderem Interesse ist die Navigation in Bereichen, die sich einer optischen Kontrolle des Operateurs entziehen, weil das Instrument beispielsweise in das Innere des Objektes eingeführt wurde. Hierzu wird die Führung des Instrumentes, beispielsweise eines Katheders, in einem virtuellen 3D-Volumen vorgenommen, welches mittels eines bildgebenden Verfahrens vor oder während der Operation erzeugt wurde. Eine häufige Anwendung ist, mit Hilfe einer Röntgendiagnostikeinrichtung eine Reihe von 2D-Projektionsaufnahmen bekannter Projektionsgeometrie zu erzeugen und aus diesen 2D-Aufnahmen einen 3D-Volumendatensatz zu erzeugen. Der Volumendatensatz wird an ein Navigationssystem übergeben, das über ein Lageerfassungssystem für von diesem erfaßbaren Marken verfügt. Um eine Navigation mit hoher Genauigkeit möglich zu machen, wird das Koordinatensystem des Lageerfassungssystem mit dem Koordinatensystem des 3D-Volumendatensatzes abgeglichen. Dieser Vorgang wird üblicherweise "Registrierung" genannt. Bei der Registrierung wird beispielsweise ein Phantom, das röntgenpositive Marken und vom Lageerfassungssystem erfaßbare Marken in einer festen Raumbeziehung zueinander enthält, verwendet. Zur Verbesserung der Genauigkeit eines rekonstruierten 3D-Datensatzes aus 2D-Röntgenprojektionsaufnahmen sind Verfahren bekannt, die die Abweichungen der Parameter der Prokjektionsgeometrie von der realen, beispielsweise durch mechanische Verwindungen der Röntgendiagnostikeinrichtung beeinflußten Projektionsgeometrie berücksichtigt. Hierzu wird eine Röntgendiagnostikeinrichtung unter Verwendung spezieller Röntgenphantome "kalibriert". Eine solche Kalibrierung erfolgt in der Regel lediglich vor der Auslieferung, nach einer Reparatur mit Austausch mechanischer Komponenten oder vor Beginn einer Untersuchung.

Aus der DE 102 02 091 A1 ist eine Vorrichtung und ein Verfahren zur Ermittlung einer Koordinatentransformation unter Verwendung eines Phantoms bekannt, an dem röntgenpositive Marken und von einem Lageerfassungssystem erfaßbare Marken in einer festen räumlichen Beziehung zueinander angeordnet sind. Bei einem Scan zur Erzeugung von 2D-Röntgenprojektionsaufnahmen werden die Koordinaten der röntgenpositiven Marken im rekonstruierten 3D-Volumen ermittelt und zum Abgleich an das Lageerfassungs- und Navigationssystem übermittelt.

Aus der US-Patentschrift US 5 442 674 A und aus der deutschen Patentschrift DE 199 47 382 C2 sind Röntgenphantome bekannt, mittels derer die mechanischen Unzulänglichkeiten der Röntgendiagnostikeinrichtung in einem Kalibriervorgang außerhalb eines Operationseinsatzes durchgeführt wird.

Die US-Patentschrift US 5 835 563 A betrifft ein Röntgenphantom, das während einer Röntgenuntersuchung fest mit dem Patienten verbunden bleibt und die Genauigkeit der Darstellungen bei der Digitalen Substraktions-Angiografie (DAS) verbessert.

Aufgabe der Erfindung ist es, die Volumenrekonstruktion unter Verwendung einer Röntgendiagnostikeinrichtung und eines Lageerfassungssystems auf einfache und kostengünstige Weise zu verbessern.

Die Aufgabe der Erfindung wird durch eine Vorrichtung gelöst, die eine Röntgendiagnostikeinrichtung mit einem Bildverarbeitungsrechner, ein Lageerfassungssystem und ein mit dem Untersuchungsobjekt verbundenes Phantom mit röntgenpositiven Marken und vom Lageerfassungssystem erfaßbaren Marken aufweist, wobei die Röntgendiagnostikeinrichtung dafür ausgelegt ist, einen Volumendatensatz des zu untersuchenden Bereiches zu erzeugen. Während eines Scans zur Aufnahme einer Reihe von 2D-Projektionsaufnahmen unterschiedlicher Projektionsgeometrie für die nachfolgende 3D-Volumenrekonstruktion sind das Phantom und das Untersuchungsobjekt im Raum fixiert. Die Positionen der röntgenpositiven Marken in den 2D-Röntgenprojektionsaufnahmen werden mittels eines Auswerteprogrammes im Bildverarbeitungsrechner der Röntgendiagnostikeinrichtung bestimmt und daraus eine Vorschrift für die Transformation eines jeden einzelnen 2D-Datensatzes ermittelt. Die transformierten 2D-Datensätze werden zur Rekonstruktion eines Volumendatensatzes herangezogen und an das Lageerfassungssystem übermittelt. Die Navigation eines Instrumentes erfolgt nach diesem Verfahren in einem rekonstruierten Volumen hoher Genauigkeit, dessen Datensatz aus einer Reihe von überlagerten 2D-Röntgenprojektionen des Objektes und des Phantoms errechnet wird. Aus dem Vergleich der 2D-Projektion des Phantoms mit den in der Röntgendiagnostikeinrichtung gespeicherten Daten für die Kinematik wird nach bekannten Verfahren jede 2D-Projektion transformiert. Die Transformation besteht im einfachsten Fall in einer Verschiebung der originalen 2D-Projektion um einen Verschiebevektor in der Ebene des Eintrittsfensters des Röntgenstrahlenempfängers. Für die Rückberechnung der Projektionsgeometrie kann es auf Grund der Beschaffenheit des Phantoms notwendig oder sinnvoll sein, bestimmte Annahmen zu treffen. So kann eine Annahme beispielsweise lauten: die Position des Eingangsfensters des Röntgenstrahlenempfängers wird unter Berücksichtigung der in einem Kalibrierlauf gewonnenen Korrekturtabellen aus der Kinematik bestimmt und die Verschiebungen des Abbildes des Phantoms in der 2D-Projektion wird ausschließlich der Verschiebung des Fokuspunktes der Röntgenstrahlenquelle zugeschrieben. Eine andere Annahme könnte lauten: die Abweichungen der 2D-Projektion des Phantoms von dem aus der Kinematik errechneten Wertes wird zu gleichen Teilen auf den Röntgenstrahlenempfänger und auf die Röntgenstrahlenquelle aufgeteilt. Im Rahmen der Erfindung ist es weiterhin vorgesehen, die Aufteilung des Betrages der Abweichung der Positionen der Röntgenstrahlenquelle und des Röntgenstrahlenempfängers abhängig von den Einstellungen der Achsen der Röntgendiagnostikeinrichtung in Wertetabellen des Bildverarbeitungsrechners zu hinterlegen.

Je mehr röntgenpositive Marken im Operationsfeld erfaßt werden, um so komplexere Transformationen können zugelassen werden; beispielsweise Bildvergrößerung, Bildverzerrung oder dergleichen. Die Praxis hat gezeigt, daß alleine schon die Berücksichtigung einer einzelnen röntgenpositiven Marke für die Transformation der 2D-Projektionen eine wesentliche Verbesserung der Güte des 3D-Datensatzes ergibt.

Die Erfindung wird an Hand der Abbildung näher erläutert.

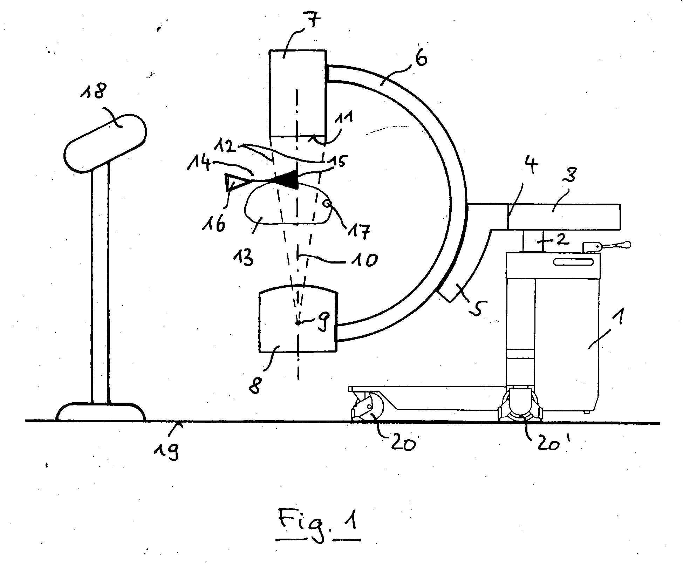

In Fig. 1 ist eine mobile Röntgendiagnostikeinrichtung mit einem auf Rollen 20, 20' längs des Fußbodens 19 verschiebbaren Gerätewagen 1 der einen mehrfach verstellbaren C-Bogen 6 trägt. Die Röntgenstrahlenquelle 8 und der Röntgenstrahlenempfänger 7 sind an den Enden eines C-Bogens 6, der längs seines Umfanges in einer C-Bogenhalterung 5 um den Mittelpunkt 17 verschieblich gelagert ist, angeordnet. Die C-Bogenhalterung 5 ist an dem Gerätewagen 1 mehrfach verstellbar angeordnet. Die C-Bogenhalterung 5 ist mit einem Schwenklager 4 an einer Horizontalführung 3 um eine horizontale Achse schwenkbar gelagert. Die Horizontalführung 3 ist an einer Säule 2 höhenverstellbar und um die senkrechte Achse der Säule 2 drehbar gelagert. Es sind vorzugsweise alle Verstelleinrichtungen des C-Bogens 6 sind mit Positionsmeßsensoren ausgerüstet, deren Meßwerte einer zentralen Bewegungssteuerung der Röntgendiagnostikeinrichtung zugeführt werden. Es ist vorgesehen, alle Verstellachsen wahlweise einzeln oder in der Gesamtheit durch Bremsen arretierbar auszuführen. Insbesondere ist es vorgesehen, die Rollen 20, 20' mit einer Feststellbremse auszurüsten. Vorzugsweise sind die Verstellbewegung des C-Bogens in der Halterung (Orbitalbewegung), die Verstellung in der Horizontalführung 3 und die vertikale Verstellung in der Säule elektromotorisch verstellbar ausgeführt, wobei die in den Verstellachsen angeordneten Motoren von einer zentralen Bewegungssteuerung der Röntgendiagnostikeinrichtung gesteuert werden.

Das Lageerfassungssystem kann ein optisches(Infrarotsystem), elektromagnetisches,: oder ein auf der Vermessung eines Magnetfeldes beruhendes System sein.

Die Genauigkeit der Navigation mit einem Instrument, das Marken enthält, die von dem Lageerfassungssystem erfaßt werden wird durch die Verwendung von transformierten 2D-Datensätzen zur Volumenrekonstruktion verbessert.

### Bezugszeichenliste:

- 1: Gerätewagen
- 2: Säule
- 3: Horizontalführung
- 4: Schwenklager
- 5: C-Bogenhalterung
- 6: C-Bogen
- 7: Röntgenstrahlenempfänger
- 8: Röntgenstrahlenquelle
- 9: Brennfleck
- 10: Zentralstrahl
- 11: Eingangsfenster
- 12: Strahlenkegel
- 13: Untersuchungsobjekt
- 14: Phantom
- 15: Röntgenpositive Marken
- 16: Marken
- 17: Mittelpunkt des C-Bogens
- 18: Lageerfassungssystem
- 19: Fußboden
- 20, 20': Rolle

## Patentansprüche

1. Vorrichtung zur Verfügungstellung einer verbesserten Rekonstruktion eines Volumens bei navigationsgeführten Eingriffen an einem Untersuchungsobjekt umfassend eine Röntgendiagnostikeinrichtung mit einem Bildverarbeitungsrechner und einem Phantom, das in einer festen räumlichen Beziehung röntgenpositive Marken und solche enthält, die von einem Lageerfassungssystem erfassbar sind wobei
- die Röntgendiagnostikeinrichtung ausgelegt ist, um eine Reihe von 2D-Projektionsaufnahmen des zu untersuchenden Bereiches mit unterschiedlichen Projektionsgeometrien zu erzeugen, wobei jede Projektion wenigstens einen Teil der röntgenpositiven Marken des Phantoms enthält,
- das Phantom so konfiguriert ist, dass es an dem Untersuchungsobjekt im zu untersuchenden Bereich angeordnet ist, während des gesamten Eingriffes mit diesem starr verbunden ist und während der Aufnahme der Reihe der 2D-Projektionsaufnahmen raumfest bleibt,
**dadurch gekennzeichnet, dass**
- der Bildverarbeitungsrechner konfiguriert ist, um die Positionen der röntgenpositiven Marken aus den 2D-Projektionsaufnahmen zu bestimmen, mit den errechneten Sollpositionen der röntgenpositiven Marken zu vergleichen, jede 2D-Projektionsaufnahme mit sich selbst zu kalibrieren und derart zu transformieren, dass die Summe der Abweichungsbeträge zwischen den Positionen der Marken in den aufgenommenen und in den transformierten 2D-Projektionsaufnahmen minimal ist,
wobei die Transformation auf der Annahme beruht, dass die Position des Eingangsfensters des Röntgenstrahlenempfängers der Röntgendiagnostikeinrichtung unter Berücksichtigung der in einem Kalibrierlauf gewonnenen Korrekturtabellen aus einer Kinematik bestimmt ist und die Abweichungen ausschließlich der Verschiebung des Fokuspunktes der Röntgenstrahlenquelle der Röntgendiagnostikeinrichtung zugeschrieben werden, und
- der Bildverarbeitungsrechner weiterhin konfiguriert ist, die Volumenrekonstruktion mit den transformierten 2D-Projektionsaufnahmen durchzuführen.

2. Vorrichtung nach Anspruch 1,
**dadurch gekennzeichnet, dass** die Transformation in einer Verschiebung der aufgenommenen 2D-Projektionsaufnahmen um einen Verschiebevektor in der Ebene des Eintrittsfensters des Röntgenstrahlenempfängers besteht.

3. Vorrichtung nach einem der Ansprüche 1-2,
**dadurch gekennzeichnet, dass** die Vorrichtung weiterhin ein Lageerfassungssystem aufweist und der Bildverarbeitungsrechner konfiguriert ist, um das rekonstruierte Volumen, bezogen auf ein mit den röntgenpositiven Marken des Phantoms verbundenes Koordinatensystem an das Lageerfassungssystem zu übermitteln.

## Claims

1. A device for providing improved volume reconstruction in navigationally guided operations on an examination subject comprising an X-ray diagnostic apparatus with an image-processing computer and a phantom containing X-ray-positive marks and marks detectable by a position detection system in a fixed spatial relation to each other, wherein
- the X-ray diagnostic apparatus is designed to generate a series of 2D projection images of the area to be examined with different projection geometries, each projection containing at least a part of the X-ray-positive marks of the phantom,
- the phantom is configured so that it is positioned on the examination object in the area to be examined, is rigidly connected to it during the entire operation, and remains fixed in space during the acquisition of the series of 2D projection images,
**is characterized in that**
- the image-processing computer is configured to determine the positions of the X-ray-positive marks from the 2D projection images, to compare them with the calculated nominal positions of the X-ray-positive marks, to calibrate each 2D projection image with itself and to transform them to the extent that the sum of the deviation amounts between the two positions of the marks in the recorded and in the transformed 2D projection images is minimal, the transformation being based on the assumption that the position of the X-ray receiver entry window of the X-ray diagnostic apparatus is determined taking the kinematics correction tables obtained in a calibration run and the deviations exclusively attributed to the displacement of the focal spot of the X-ray source of the X-ray diagnostic apparatus into account, and
- the image-processing computer is further configured to perform the volume reconstruction with the transformed 2D projection images.

2. Device according to claim 1,
**characterized in that** the transformation consists in the displacement of the recorded 2D projection images by a displacement vector in the plane of the entry window of the X-ray receiver.

3. Device according to one of claims 1-2,
**characterized in that** the device further comprises a position detection system and the image-processing computer is configured to relay the reconstructed volume to the position detection system relative to a system of coordinates connected to the x-ray-positive marks of the phantom.

## Revendications

1. Dispositif mettant à disposition une reconstruction améliorée d'un volume lors des interventions guidées par navigation réalisées sur un objet d'examen qui comprennent un dispositif de diagnostic à rayons X pourvu d'un ordinateur de traitement d'images et d'un fantôme qui comporte, dans une relation spatiale fixe, des marques positives aux rayons X ainsi que de telles marques qui peuvent être détectées par un système de détection de position, dans la mesure où :
- le dispositif de diagnostic à rayons X est conçu pour générer une série d'images de projections 2D de la zone à examiner avec différentes géométries de projection et que chaque projection comporte au moins une partie de la marque positive aux rayons X du fantôme ;
- le fantôme est configuré de sorte qu'il est situé sur l'objet de l'examen, dans la zone à examiner, qu'il est connecté avec celui-ci de manière fixe tout au long de l'intervention et qu'il reste immobile dans l'espace pendant l'acquisition de la série d'images de projection 2D ;
**se caractérisant par le fait que :**
- l'ordinateur de traitement d'images est configuré pour déterminer les positions des marques positives aux rayons X des images de projection 2D, pour les comparer aux positions nominales calculées des marques positives aux rayons X, pour calibrer chaque image de projection 2D avec elle-même et pour les transformer, de telle manière que la somme des écarts entre les positions des marques dans les images de projection 2D prises et dans celles transformées soit minimale, à condition que la transformation repose sur l'hypothèse que la position de la fenêtre d'entrée du récepteur de rayons X du dispositif de diagnostic à rayons X est déterminée par une Kinematics, en tenant compte des tableaux de correction recueillis pendant un essai d'étalonnage, et que les écarts sont exclusivement imputables au décalage du point focal de la source de rayons X du dispositif de diagnostic à rayons X ; et **le fait que**
- l'ordinateur de traitement d'images est en outre configuré pour effectuer la reconstruction de volumes à l'aide des images de projection 2D transformées.

2. Dispositif selon la revendication 1,
**se caractérisant par le fait que** la transformation consiste en un décalage des images de projection 2D prises autour d'un vecteur de décalage, au niveau de la fenêtre d'entrée du récepteur de rayons X.

3. Dispositif selon l'une des revendications 1 - 2,
**se caractérisant par le fait que** le dispositif présente en outre un système de détection de position et que l'ordinateur de traitement d'images est configuré pour transmettre au système de détection de position le volume reconstruit par rapport à un système de coordonnées relié aux marques positives aux rayons X du fantôme.
